# EUROPEAN PATENT APPLICATION

(11) **EP 1 329 452 A1**
(43) Date of publication of application: **23.07.2003**
(21) Application number: 01976774.8
(22) Date of filing: 19.10.2001
(51) Int. Cl.: C07D 493/18, A61K 31/357, A61K 31/737, A61P 33/06

(54) **MEDICINAL COMPOSITIONS, DOSE AND METHOD FOR TREATING MALARIA**

(30) Priority: 20.10.2000 JP 2000321022
(71) Applicant: Ajinomoto Co., Inc., Tokyo 104-8315 (JP); University of the Witwatersrand, Johannesburg 2193 (ZA)
(72) Inventor: KANEKO, Yutaro, c/o AJINOMOTO CO., INC., Tokyo 104-8315 (JP); HAVLIK, Ivan, c/o Medical School, Johannesburg 2193 (ZA)
(74) Representative: Nicholls, Kathryn Margaret
(86) International application number: JP0109199
(87) International publication number: WO02032905

(57) **Abstract**

Novel pharmaceutical compositions, unit dose systems and methods for the treatment of malaria are provided whereby the antimalarial activity of sulfated polysaccharides can be enhanced. The antimalarial activity of the sulfated polysaccharides was remarkably enhanced when the sulfated polysaccharides and artemisinin or artemisinin dihydro derivative were administered in combination.

## Description

### Technical Field

The present invention relates to pharmaceutical compositions, unit dose systems and methods for the treatment of malaria. Specifically, the present invention relates to pharmaceutical compositions and unit dose systems combining a sulfated polysaccharide and artemisinin or an artemisinin dihydro derivative (artemisinin and artemisinin dihydro derivatives are collectively called "artemisinin compounds") and methods for treating malaria by combining a sulfated polysaccharide and an artemisinin compound.

### Background Art

Malaria is a serious health care problem posing a great menace to us in both of the number of patients and the mortality of patients as evidenced by about 500 million patients attacked annually predominantly in tropical and subtropical regions.

Malaria is treated by administering chloroquine, pyrimethamine, quinine, proguanil, primaquine, artemisinin compounds, etc.

However, effective treatments have become difficult with conventional antimalarial drugs as malarial parasites having resistance to these antimalarial drugs (e.g. *Plasmodium falciparum*) are recently rapidly increasing.

Sulfated polysaccharides are known to have anti-retroviral activity (JPA S62-215529) or antimalarial activity (WO95/08334).

Artemisinin is a sesquiterpene lactone having the structure of formula II:

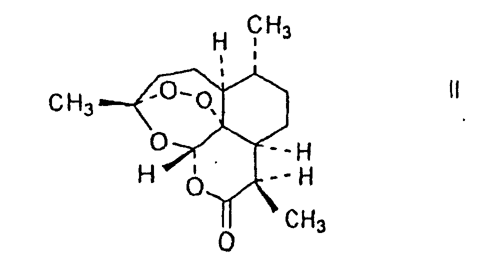

This compound was isolated from a medicinal herb *Artemisia annula L. Compositae* used in China since ancient times and is known to have antimalarial activity (Chinese Med. J. 92, 811 (1979)). Artemisinin dihydro derivatives having the structure of formula I: 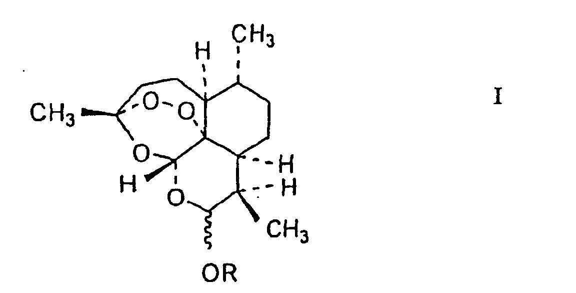 are also known to have antimalarial activity, wherein R represents H (dihydroartemisinin), CH₃ (artemether), CH₂CH₃ (arteether), CH₂C₆H₄COOM (artelinate) or COCH₂CH₂COOM (artesunate) where M represents H or a cation that may replace H in the carboxyl group to form a salt

Given the seriousness of the menace of malaria, novel pharmaceutical compositions, unit dose systems and methods for the treatment of malaria that are effective against parasites having resistance to conventional antimalarial drugs would be desirable.

Accordingly, the present invention aims to provide novel pharmaceutical compositions, unit dose systems and methods for the treatment of malaria by enhancing the antimalarial activity of sulfated polysaccharides. These novel pharmaceutical compositions, unit dose systems and methods are effective for the treatment of malaria caused by parasites having resistance to conventional antimalarial drugs.

### Disclosure of the Invention

We have continued our studies to enhance the antimalarial activity of sulfated polysaccharides which we had previously found to have an antimalarial mechanism potentially distinct from those of conventional antimalarial drugs. Then, we found that the antimalarial activity of these sulfated polysaccharides was remarkably enhanced when they were combined with artemisinin compounds. We also found that these sulfated polysaccharides had an enhanced therapeutic effect against malaria caused by parasites resistant to conventional antimalarial drugs when they were combined with artemisinin compounds.

Accordingly, a first aspect of the present invention is a pharmaceutical composition to be administered to patients in need of the treatment of malaria, comprising a sulfated polysaccharide and an artemisinin compound each in an effective amount for the treatment of malaria in combination with the other as well as a pharmaceutically acceptable carrier. The sulfated polysaccharide here is more preferably curdlan sulfate.

A second aspect of the present invention is a unit dose system comprising a combination of a unit dose of a pharmaceutical composition to be administered to patients in need of the treatment of malaria containing a sulfated polysaccharide in an effective amount for the treatment of malaria in combination with an artemisinin compound and a pharmaceutically acceptable carrier and a unit dose of a pharmaceutical composition to be administered to patients in need of the treatment of malaria containing said artemisinin compound in an effective amount for the treatment of malaria in combination with said sulfated polysaccharide and a pharmaceutically acceptable carrier. The sulfated polysaccharide here is more preferably curdlan sulfate.

Another aspect of the present invention is a method for treating malaria, comprising administering to patients in need of the treatment of malaria a combination of a sulfated polysaccharide and an artemisinin compound each in an effective amount for the treatment of malaria in combination with the other.

The sulfated polysaccharide here is more preferably curdlan sulfate.

### Brief Explanation of the Drawings

FIG. 1 shows test results of recovery from fever by combined administration of curdlan sulfate (CRDS) and artesunate. Solid line shows CRDS + artesunate, and dotted line shows placebo + artesunate.

FIG. 2 shows test results of recovery from coma by combined administration of curdlan sulfate (CRDS) and artesunate. Solid line shows CRDS + artesunate, and dotted line shows placebo + artesunate.

### Detailed Description of the Invention

As used herein, "sulfated polysaccharides" have a sulfate moiety in their polysaccharide molecule. The sulfate moiety may be attached to the hydroxyl group of a sugar by an ester linkage (O-sulfate) or to the amino group of an amino sugar (N-sulfate).

Sulfated polysaccharides having O-sulfate groups include those naturally occurring and those obtained by artificially introducing O-sulfate groups.

Examples of naturally occurring sulfated polysaccharides having O-sulfate groups include chondroitin sulfates A, B, C, D, E, H and K, dermatan sulfate and keratan sulfate.

Sulfated polysaccharides having artificially introduced O-sulfate groups are more preferred in the present invention. The sulfated polysaccharides having artificially introduced O-sulfate groups may have any polysaccharide moiety, e.g. it may consist essentially of pentose residues (pentosans) or hexose residues.

Well known pentosans include arabinans and xylans.

Polysaccharides consisting essentially of hexose residues are most preferably glucans consisting essentially of glucose residues.

Glucose units of glucans may be joined by α-glycosidic linkages or β-glycosidic linkages or mixtures of α- and β-glycosidic linkages. The glycosidic linkages may occur at any positions including 1-3, 1-4 and 1-6 linkages.

More specifically, glucans include dextrans mainly containing α-1,6 linkages (which give a sulfated polysaccharide known as dextran sulfate), dextrin mainly containing α-1,4 linkages and pullulan containing mixed α-1,4 and α-1,6 linkages.

In the present invention, the most preferred polysaccharide moieties of sulfated polysaccharides are β-glucans mainly containing β-glycosidic linkages. β-Glucans include those mainly containing β-1,4 glycosidic linkages (cellulose), those mainly containing β-1,6 glycosidic linkages and those mainly containing β-1,3 glycosidic linkages. The most preferred β-glucans in the present invention mainly contain β-1,3 glycosidic linkages.

Polysaccharides often contain different linkages (α-linkages, β-linkages, different positions of linkage) as both α- and β-glucans were described above by the term "essentially" or "mainly". Even glucans, for example, sometimes contain minor amounts of other sugar units than glucose.

Examples of glucans mainly containing β-1,3 glycosidic linkages, which are the most preferred polysaccharides in the present invention, include curdlan, succinoglucan, lentinan, schizophyllan, sclerotan and lutean. Among these glucans having β-1,3 glycosidic linkages, curdlan and lentinan are the most preferred.

Sulfated polysaccharides having N-sulfate groups also include those naturally occurring (e.g. heparin) and those obtained by artificially introducing an N-sulfate group into the amino group of an amino sugar such as those having a sulfate group introduced into the amino group of chitosan.

Sulfated polysaccharides of the present invention also include modified polysaccharides such as carboxymethylated polysaccharides.

Well known methods can be applied to introduce O-sulfate or N-sulfate groups into polysaccharides. The sulfur content in sulfated polysaccharides is 5-25 % by weight, preferably 10-20 % by weight, especially 12-17 % by weight.

The sulfated polysaccharides have a molecular weight of 5,000-500,000, preferably 20,000-200,000, especially 50,000-120,000 as an average molecular weight measured by gel permeation chromatography. Generally, retentivity in blood is higher as the molecular weight is higher.

Sulfated polysaccharides are preferably formulated and administered in the form of pharmaceutically acceptable salts. The most preferred salts are alkali metal salts or alkali earth metal salts (sodium salts, potassium salts, calcium salts and magnesium salts, etc.), and ammonium salts or nontoxic amine salts (ammonium salts, tetramethylammonium salts, tetraethylammonium salts, methylammonium salts, dimethylammonium salts, trimethylammonium salts, triethylammonium salts, diethylammonium salts, ethylammonium salts, lysine salts, arginine salts, ethylenediamine salts, ethanolamine salts, diethanolamine salts, piperidine salts and piperazine salts).

In artemisinin dihydro derivatives of formula I, M is most preferably Na. Artemisinin dihydro derivatives may exist as α-isomers and β-isomers depending on the position of the OR group attached.

Pharmaceutical compositions of the present invention contain a pharmaceutically acceptable carrier in addition to a sulfated polysaccharide and an artemisinin compound. The pharmaceutically acceptable carrier depends on the dosage form.

When the pharmaceutical compositions are used for oral administration, they may appropriately contain pharmaceutically acceptable carriers including binders such as gum tragacanth, gum arabic, corn starch and gelatin; excipients such as dicalcium phosphate; disintegrants such as potato starch and alginic acid; lubricants such as magnesium stearate; sweetening agents such as sucrose; dyes; and perfumes such as orange flavor; and solvents such as water, ethanol and glycerol.

When pharmaceutical compositions of the present invention are injectable compositions, suitable pharmaceutically acceptable carriers include sterilized water, isotonic saline and pH buffers. Alternatively, injectable compositions of the present invention may be sterilized powder compositions or lyophilized powder compositions that can be used by simple dissolution in sterilized water. Injectable pharmaceutical compositions of the present invention may contain sugars (glucose, mannitol and dextran, etc.), polyhydric alcohols (glycerol, etc.), and inorganic salts (sodium salts and magnesium salts, etc.).

When pharmaceutical compositions of the present invention are administered by intravenous infusion, they may contain nutrients such as glucose, vitamins, amino acids and lipids.

Pharmaceutical carriers to be added to dosage forms for other administration modes such as nasal administration, inhalation and transdermal administration are also well-known to those skilled in the art.

When pharmaceutical compositions of the present invention are orally administered, they may be in the form of controlled- or sustained-release formulations. Well-known sustained-release formulations include ordinary sustained- or controlled-release formulations such as gel-coated formulations and multicoated formulations as well as site-specific delivery formulations (e.g. burst release at pyloric regions or effervescent delivery to the duodenum). Oral compositions include, for example, tablets, pills, capsules, ampoules, sachets, elixirs, suspensions, syrups, etc.

The dosage forms and pharmaceutical carriers mentioned above are described in Remington's Pharmaceutical Sciences, 16th ed. (1980), Mack Publishing Company, which is incorporated herein as reference.

A sulfated polysaccharide and an artemisinin compound may be administered to patients with malaria as a pharmaceutical composition of the present invention in a single formulation, or a unit dose of a pharmaceutical composition containing a sulfated polysaccharide and a unit dose of a pharmaceutical composition containing an artemisinin compound may be administered to patients with malaria at an interval in any order.

That is, a unit dose system comprising a combination of a unit dose of a pharmaceutical composition containing a sulfated polysaccharide and a unit dose of a pharmaceutical composition containing an artemisinin compound is also included in one aspect of the present invention.

As used herein, the "unit dose" includes not only individually packaged unit doses such as vials but also aliquots dispensed from vials into syringes and compositions for infusion contained in infusion containers.

In the "unit dose system" comprising a combination of two unit doses according to the present invention, the two unit doses may be combined in a single package or spatially and/or temporally separated. For example, one unit dose may be a vial for injection (or an injectable composition contained in a syringe), and the other may be a unit dose to be orally administered at a time interval after administration of the former unit dose.

In methods for treating malaria of the present invention, a sulfated polysaccharide and an artemisinin compound are administered in combination.

In the combination of a sulfated polysaccharide and an artemisinin compound, the sulfated polysaccharide and the artemisinin compound may be administered to patients simultaneously or separately at a time interval in any order.

Pharmaceutical compositions or unit dose systems of the present invention can be administered via various routes such as transmucosal (sublingual, nasal, buccal), oral, enteral, transdermal or intravenous administration, inhalation, suppositories or intravenous infusion. These administration modes depend on the amount of the sulfated polysaccharide to be administered, the condition of the patient and other factors. Among these administration modes, intravenous administration especially by injection and infusion is most preferred.

The administration routes of a sulfated polysaccharide (or a unit dose of a pharmaceutical composition containing a sulfated polysaccharide) and an artemisinin compound (or a unit dose of a pharmaceutical composition containing an artemisinin compound) may be the same or different. For example, a sulfated polysaccharide may be administered as an intravenous bolus and an artemisinin compound may be orally administered.

In the present invention, the effective amount of sulfated polysaccharides used for the treatment of malaria in combination with artemisinin compounds is normally 1-1,000 mg/kg weight daily, preferably 5-500 mg/kg weight daily depending on the age, body weight and condition of the patient and the administration mode.

In the present invention, the effective amount of artemisinin compounds used for the treatment of malaria in combination with sulfated polysaccharides is normally 1-5,000 mg/kg weight daily, preferably 100-3000 mg/kg weight daily depending on the age, body weight and condition of the patient and the administration mode.

### EXAMPLES

### Example 1

This example demonstrates that the fever in patients with severe malaria is more effectively eliminated by combining a sulfated polysaccharide and an artemisinin compound.

Sodium curdlan sulfate (hereinafter referred to as "CRDS"; molecular weight 80,000; sulfur content 14.5 %) was used as a sulfated polysaccharide and artesunate (dihydroartemisinin hemisuccinate, see formula I, J. Med. Chem., 30, 2147 (1987)) was used as an artemisinin compound.

CRDS was used as a solution containing 100 mg of CRDS, 50 mg of mannitol, 18 mg of dibasic sodium phosphate and phosphate buffer (pH 6.5) per vial.

CRDS was intravenously administered (infusion) at 4 mg/kg every 8 hours in a total daily dose of 12 mg/kg for 4 days.

Physiological saline was used as a placebo and administered according to the same schedule as for CRDS.

Artesunate (60 mg/vial) was intravenously administered to both CRDS and placebo groups at a dose of initially 120 mg, and then 60 mg every 12 hours up to a total dose of 600 mg (according to the current standard therapy in the Bangkok Hospital for Tropical Diseases). If possible, tablets containing 50 mg of artesunate were orally administered.

Fifty patients were randomly divided into a CRDS group and a placebo group. The patients were males and females at the ages of 12-65 in whom *P. falciparum* was found. The patients had symptoms of severe malaria (with hypoglycemia corrected) and excluded those having symptoms of cerebral malaria.

The febrile state was observed in the CRDS group and the placebo group. The patients who departed from this protocol were excluded, and the observation of the febrile state was completed on 22 patients in the CRDS group and 22 patients in the placebo group.

The time required for each patient to recover from fever after starting the treatment was recorded. Fisher's exact test was used to compare the number of patients who recovered from fever within 120 hours between both groups (CRDS group and placebo group).

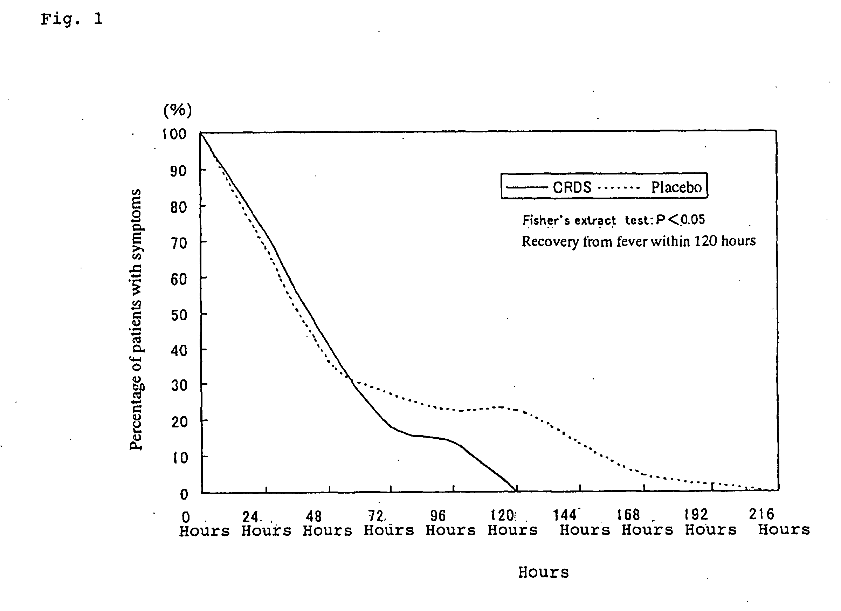

The results are shown in FIG. 1. As shown from FIG. 1, all the patients in the CRDS group (combined with artesunate) recovered from fever within 5 days, as compared with the placebo group (artesunate alone) in which 9 days were required for all the patients to recover from fever. That is, combined administration of CRDS and artesunate remarkably shortened recovery from fever as compared with administration of artesunate alone. The result of the Fisher's test was P < 0.05.

No recurrence of malaria was found for up to 28 days of observation.

### Example 2

This example demonstrates that the come induced by malaria is more quickly eliminated by administering CRDS and artesunate in combination as compared with artesunate alone.

CRDS (molecular weight 80,000; sulfur content 14.5 %) was used as a sulfated polysaccharide and artesunate was used as an artemisinin compound.

CRDS was used as a solution containing 100 mg of CRDS, 50 mg of mannitol, 18 mg of dibasic sodium phosphate and phosphate buffer (pH 6.5) per vial.

CRDS was intravenously administered (infusion) at 4 mg/kg every 8 hours in a total daily dose of 12 mg/kg for 4 days.

Physiological saline was used as a placebo and administered according to the same schedule as for CRDS.

Artesunate (60 mg/vial) was intravenously administered to both CRDS and placebo groups at a dose of initially 120 mg, and then 60 mg every 12 hours up to a total dose of 600 mg. If possible, tablets containing 50 mg of artesunate were orally administered.

Thirty male patients at the ages of 18-65 in a coma were sampled who manifested symptoms of severe/cerebral malaria (with hypoglycemia corrected) as defined by the WHO criteria and also showed asexual malarial parasites and P-site test positive.

Coma was determined by Glasgow Coma Score.

For each of the CRDS group and the placebo group, 13 patients completed this protocol. The time required for each patient to recover from coma was recorded. Fisher's exact test was used to compare the number of patients who recovered from coma within 120 hours between both groups (CRDS group and placebo group).

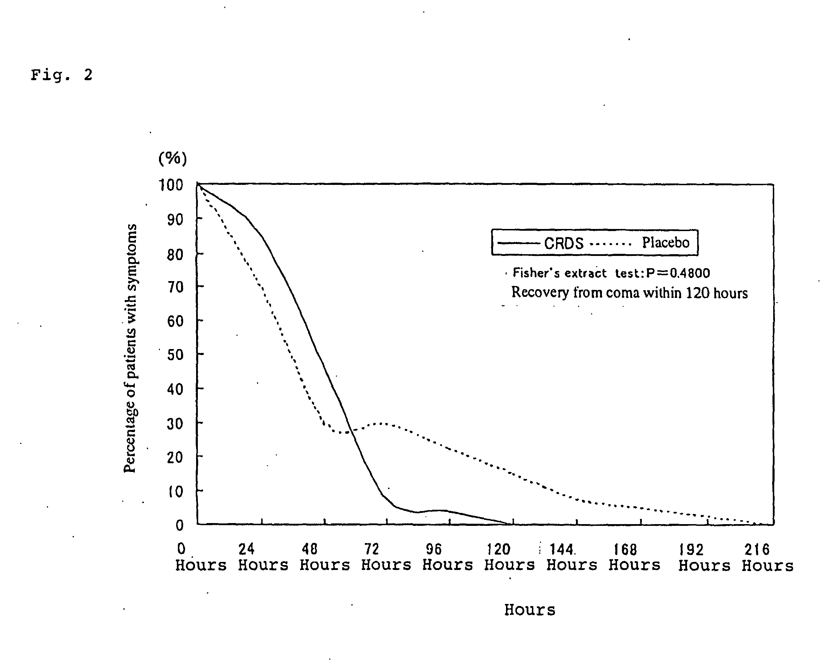

The results are shown in FIG. 2. As shown from FIG. 2, all the patients in CRDS group (combined with artesunate) recovered from coma within 5 days. However, 9 days were required for all the patients to recover from coma in the placebo group (artesunate alone). The result of the Fisher's test was 0.4800.

No recurrence of malaria was found for up to 28 days of observation.

As shown above, combined administration of CRDS and artesunate remarkably shortened recovery from the coma induced by cerebral malaria as compared with administration of artesunate alone in the same manner as in Example 1, though no significant difference was found.

## Claims

1. A medicine for the treatment of malaria, which comprises a sulfated polysaccharide and an artemisinin compound selected from a group consisting of artemisinin and artemisinin dihydro derivatives defined by the formula I: 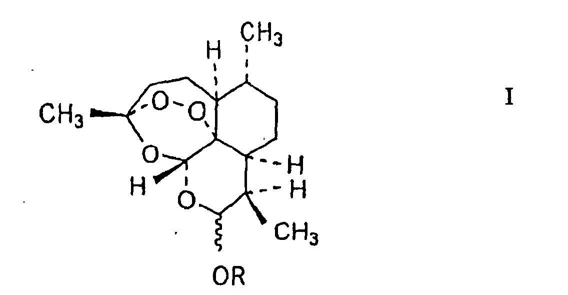 (wherein R is H, CH₃, CH₂CH₃, CH₂C₆H₄COOM or COCH₂CH₂COOM, M represents H or a cation that may replace H in the carboxyl group to form a salt).

2. A medicine for the treatment of malaria, which consists of a pharmaceutical unit comprising a sulfated polysaccharide and a pharmaceutical unit comprising an artemisinin compound selected from a group consisting of artemisinin and artemisinin dihydro derivatives defined by the formula I: 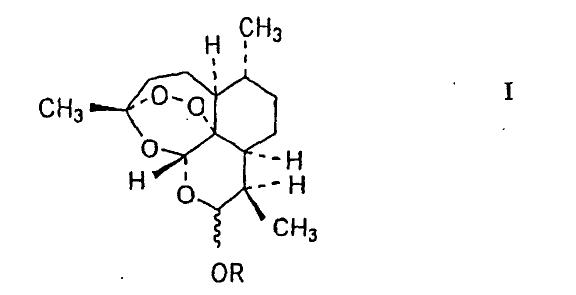 (wherein R is H, CH₃, CH₂CH₃, CH₂C₆H₄COOM or COCH₂CH₂COOM, M represents H or a cation that may replace H in the carboxyl group to form a salt).

3. A medicine according to claim 1 or 2, wherein the sulfated polysaccharide is sulfated curdlan.

4. A Method for the treatment of malaria, which comprises administrating sulfated polysaccharide and artemisinin compound selected from a group consisted of artemisinin and artemisinin dihydro derivatives defined by the formula I: 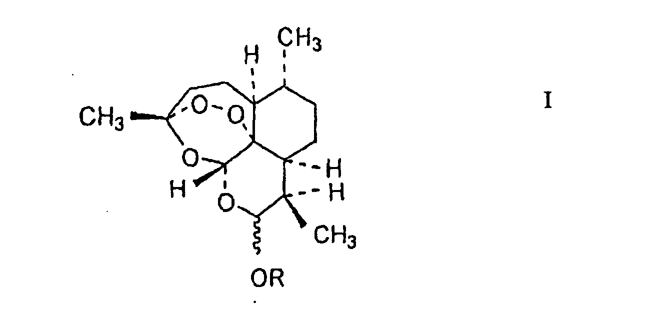 (wherein R is H, CH₃, CH₂CH₃, CH₂C₆H₄COOM or COCH₂CH₂COOM, M represents H or a cation that may replace H in the carboxyl group to form a salt).

5. A method for the treatment of malaria according to claim 4, wherein the sulfated polysaccharide is sulfated curdlan.
